# EUROPEAN PATENT APPLICATION

(11) **EP 4 166 188 A1**
(43) Date of publication of application: **19.04.2023**
(21) Application number: 22201589.3
(22) Date of filing: 14.10.2022
(51) Int. Cl.: A61N 1/32, A61N 1/08, A61B 5/01

(54) **TREATMENT APPARATUS USING RF ENERGY**

(30) Priority: 15.10.2021 KR 20210137669
(71) Applicant: Lutronic Corporation, Goyang-si, Gyeonggi-do 10534 (KR)
(72) Inventor: KO, Kwang Chon, 10893 Paju-si, Gyeonggi-do (KR)
(74) Representative: Michalski Hüttermann & Partner Patentanwälte mbB

(57) **Abstract**

Disclosed are a treatment apparatus using RF energy according to the disclosure, a control method thereof, and a treatment method using the same, in which a frequency of the RF energy is adjusted according to target tissues to which energy is specifically transferred among skin tissues, and impedance matching is performed in real time, thereby having an effect on treating the tissue efficiently and accurately.

## Description

### BACKGROUND OF THE INVENTION

### Field of the Invention

The disclosure relates to a treatment apparatus using radio frequency (RF) energy, a control method thereof, and a treatment method using the same.

### Description of the Related Art

In a medical field, radio frequency (RF) energy has been widely used for the purpose of removing or excising tissue by locally applying the RF energy to heat the tissue.

However, the RF energy is also being used for the purpose of treating aged skin tissue (e.g., wrinkles, reduced elasticity, etc.) as a method of applying the RF energy to remodel the tissue has recently been developed. Besides, the RF energy is being used to break down fat cells by applying the RF energy to a wide range of a subcutis. In this way, the scope of RF energy utilization in the medical field is expanding.

Regarding a skin treatment method using such RF energy, there has been disclosed Korean Patent No. 1246980.

However, such a related art has a problem in that the penetration depth of the RF energy applied through skin is not properly controlled because skin tissues are different in electrical characteristics.

### Documents of Related Art

### Patent Document

(Patent Document 1) Korean Patent No. 1246980

### SUMMARY OF THE INVENTION

To solve the foregoing problems, an aspect of the disclosure is to provide a treatment apparatus using radio frequency (RF) energy, a control method thereof, and a treatment method using the same, in which the RF energy to be applied through skin is adjusted in consideration of skin tissues different in electrical characteristics according to penetration depths.

According to an embodiment of the disclosure, there may be provided a treatment apparatus using RF energy, including: an RF generator configured to generate the RF energy; an RF adjuster configured to adjust the RF energy; an electrode configured to noninvasively transfer the adjusted RF energy to a target tissue while being in contact with skin; and a controller configured to control the RF generator and the RF adjuster, wherein the controller adjusts a frequency of the RF energy to intensively transfer the RF energy to any one of epidermis, dermis and subcutis.

Meanwhile, the controller may control the RF adjuster to transfer the RF energy by selecting any one of three frequencies.

Meanwhile, the treatment apparatus may further include an impedance matcher provided between the RF adjuster and the electrode, wherein the controller controls the impedance matcher to perform impedance matching between the target tissue and the RF adjuster when the RF adjuster adjusts the frequency of the RF energy.

Further, the controller may control the RF adjuster to select the frequency of the RF energy substantially from among 2.28 MHz, 6.75 MHz and 13.56 MHz.

Meanwhile the electrode may be provided at an end portion of a handpiece for face treatment.

In this case, the controller may control the RF adjuster to change the frequency of the RF energy in order of 2.25 MHz, 6.75 MHz and 13.56 MHz.

Further, the treatment apparatus may further include a cooler configured to cool the electrode, wherein the controller controls the cooler to operate when a temperature of a skin surface rises above a threshold value.

Meanwhile, the electrode may be provided in a body applicator attachable to a human body.

In this case, the body applicator may include a plurality of body applicators, and the controller may control the RF adjuster to adjust the frequency of the RF energy, which is used for at least one body applicator among the plurality of body applicators, according to predetermined conditions.

Meanwhile, the controller may control the RF adjuster to adjust the frequency of the RF energy, which is used for the body applicator, in order of 2.25 MHz, 6.75 MHz and 13.56 MHz.

Meanwhile, the plurality of body applicators may be assigned with unique numbers, respectively, and the controller may control the RF adjuster to adjust the frequency of the RF energy in sequence of the body applicators according to the unique numbers.

Furthermore, the treatment apparatus may further include a vacuum pump, and a passage provided in the body applicator and configured to fluid-communicate with the vacuum pump.

In addition, there may be provided a control method of a treatment apparatus using RF energy, the control method including: generating the RF energy; adjusting a frequency of the RF energy; and transferring the adjusted RF energy to an electrode, wherein the adjusting the frequency of the RF energy includes adjusting the frequency of the RF energy to intensively transfer the RF energy to any one of epidermis, dermis and subcutis.

Meanwhile, the adjusting the frequency of the RF energy may be performed by selecting any one of three frequencies.

Further, the control method may further include performing impedance matching between impedance of the treatment apparatus and impedance of a target tissue when the frequency of the RF energy is adjusted.

Meanwhile, the adjusting the frequency of the RF energy may be performed by substantially selecting any one frequency of 2.28 MHz, 6.75 MHz and 13.56 MHz.

Further, the transferring the adjusted RF energy to the electrode may be performed by transferring the RF energy to the electrode provided in a tip of a handpiece configured to come into close contact with a face.

Meanwhile, the control method may further include cooling to drive a cooler configured to cool the electrode when a temperature of a skin surface being in close contact with the tip rises above a threshold value.

Meanwhile, the transferring the adjusted RF energy to the electrode may be performed by transferring the RF energy to the electrode provided in a plurality of body applicators attachable to a human body.

Further, the transferring the adjusted RF energy to the electrode may be performed by adjusting the frequency of the RF energy, which is used for at least one among the plurality of body applicators, according to predetermined conditions.

In addition, there may be provided a treatment method using radio frequency (RF) energy, including: attaching an electrode to skin; treating tissue by intensively transferring the RF energy to any one of epidermis, dermis and subcutis; and cutting off the transfer of the RF energy

Meanwhile, the treating the tissue may include transferring the RF energy by selecting any one of three frequencies.

Further, the treatment method may further include performing impedance matching between a treatment apparatus using the RF energy and the tissue when the frequency is changed in the treating the tissue.

Meanwhile, the treating the tissue may be performed by selecting a frequency substantially from among 2.28 MHz, 6.75 MHz and 13.56 MHz.

Further, the attaching the electrode to the skin may include attaching the electrode to the skin of a face, and the treating the tissue may include transferring the RF energy to the electrode attached to the face.

Meanwhile, the attaching the electrode to the skin may be performed by attaching a plurality of body applicators including the electrodes to a plurality of points on a human body, and the treating the tissue may be performed by transferring the RF energy to at least one among the plurality of body applicators.

As described above, there are provided a treatment apparatus using RF energy according to the disclosure, a control method thereof, and a treatment method using the same, in which the RF energy is adjusted according to penetration depths from the surface of skin, thereby having an effect on improving treatment accuracy. Further, impedance matching is performed according to the penetration depths of the RF energy, thereby having an effect on maximizing a treatment effect in a treatment area.

### BRIEF DESCRIPTION OF THE DRAWINGS

FIG. 1 shows a block diagram of a treatment apparatus using radio frequency (RF) energy according to a first embodiment of the disclosure.
FIG. 2 shows a schematic diagram of skin tissues and a circuit diagram approximating the electrical characteristics of the tissues.
FIG. 3 shows a conceptual diagram of RF energy's frequency selected for each target tissue.
FIG. 4 shows a partially enlarged perspective view of a handpiece in a treatment apparatus using RF energy according to a second embodiment of the disclosure.
FIG. 5 shows an exploded perspective view of a tip shown in FIG. 4.
Fig. 6 shows a plan view of the electrode module.
FIG. 7 shows a cross-sectional view of the tip shown in FIG. 4.
FIG. 8 shows information about heat maps and temperatures when the surface of the tip shown in FIG. 4 is cooled.
FIG. 9 shows a partially enlarged perspective view of a body applicator in a treatment apparatus using RF energy according to a third embodiment of the disclosure.
FIG. 10 shows an exploded perspective view of the body applicator shown in FIG. 9.
FIG. 11 shows a cross-sectional view of the body applicator shown in FIG. 9.
Fig. 12 shows a plan view of an electrode plate and a view showing another embodiment.
FIG. 13 shows a use state of the treatment apparatus using the RF energy according to the third embodiment of the disclosure.
FIG. 14 shows a flowchart of a control method of a treatment apparatus using RF energy according to a fourth embodiment of the disclosure.
FIG. 15 shows a flowchart of a control method of a treatment apparatus using RF energy according to a fifth embodiment of the disclosure.
FIG. 16 shows a flowchart of a control method of a treatment apparatus using RF energy according to a sixth embodiment of the disclosure.
FIG. 17 shows a flowchart of a treatment method using RF energy according to a seventh embodiment of the disclosure.
FIG. 18 shows a flowchart of a treatment method using RF energy according to an eighth embodiment of the disclosure.
FIG. 19 shows a flowchart of a treatment method using RF energy according to a ninth embodiment of the disclosure.

### DETAILED DESCRIPTION OF THE INVENTION

Hereinafter, a treatment apparatus using radio frequency (RF) energy, a control method thereof, and a treatment method using the same according to embodiments of the disclosure will be described in detail with reference to the accompanying drawings. The names of elements used in the following description may be referred to as other names in the art. However, these elements may be considered as equivalent elements in alternative embodiments as long as they have functional similarity and identity. Further, the reference numerals of the elements are provided for the convenience of description. However, the elements indicated by the reference numerals in the drawings are not limited to the scope shown in the drawings. Similarly, even though some elements in the drawings are modified in alternative embodiments, these elements are considered as equivalent elements as long as they have functional similarity and identity. Further, when elements are regarded as elements that should be naturally included at the level of those skilled in the art, descriptions thereof will be omitted.

In this specification, descriptions will be made on the premise that treatment means to improvement in wrinkles, tone and textural changes, scars and acne scarring, sagging mucosa, overall rejuvenation, hyperhidrosis, laxity, lifting, tightening, fat reduction, etc. by locally heating skin tissue.

Hereinafter, the 'tissue' refers to a set of cells that constitute skin tissue of animals including humans, and may include skin tissue of a face, a body, etc.

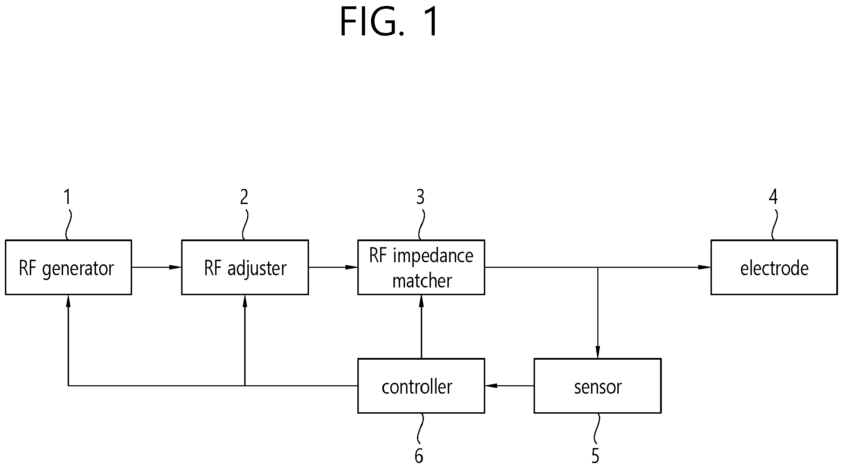

FIG. 1 shows a block diagram of a treatment apparatus using RF energy according to a first embodiment of the disclosure.

Referring to FIG. 1, an RF treatment apparatus according to a first embodiment of the disclosure is configured to heat and treat tissue by applying RF energy into the tissue. The RF treatment apparatus according to the disclosure may include an RF generator 1, an RF adjuster 2, an impedance matcher 3, an electrode 4, a sensor 5, and a controller 6.

The RF generator 1 is configured to receive power and generate RF energy. However, the RF generator may have the well-known configuration for generating the RF energy, and thus detailed descriptions thereof will be omitted.

The RF adjuster 2 is configured to generate the RF energy for the treatment through the electrode 4, while adjusting the frequency, voltage, etc. of the RF energy as necessary. The RF adjuster 2 may adjust the frequency so that a main energy-applying area can be changed when the RF energy is applied from the surface of skin to a deep part. For example, the RF adjuster 2 may be configured to adjust the frequency of the RF energy into 2.25, 6.75 and 13.56 MHz so that the RF energy can be intensively applied to the epidermis, dermis and subcutis of the skin.

The impedance matcher 3 is configured to perform impedance matching between a circuit of the treatment apparatus using the RF energy and the tissue. When the tissue is varied in temperature, the tissue's own impedance may be varied. Further, when the tissue to be treated is changed, the impedance matching may be performed in real time based on the impedance of the changed tissue. The impedance matcher 3 performs matching with an impedance treatment apparatus to maximize an energy transfer efficiency, thereby shortening time taken in the treatment and exhibiting a precise treatment effect.

The electrode 4 is configured to transfer the RF energy from the RF treatment apparatus to skin. There may be provided a plurality of electrodes 4 to transfer the energy to a plurality of points. The electrode 4 may be configured as a bipolar type so that the RF energy can be focused on a lesion positioned between each pair of electrodes. Alternatively, a plurality of pairs of electrodes 4 may be provided and configured to generate fractional damage in the tissue. The plurality of electrodes 4 may be noninvasively configured.

The sensor 5 is configured to measure the output values of the RF energy. The sensor 5 may be provided on an electrical path between the RF generator 1 and the electrode 4, and configured to measure the current, voltage, and power applied to the electrode 4. From the measured current, voltage, and power, it is possible to calculate the impedance value of the tissue.

The controller 6 is configured to receive the measured values from the sensor 5, and control the RF generator 1 and the like. Further, the controller 6 is configured to control the RF generator 1, the RF adjuster 2, the impedance matcher 3, and the like elements during operation based on a user's input. The controller 6 may identify condition inside the tissue while applying the RF energy and function to adjust the applied RF energy or cut off the RF energy. In this case, when the target tissue to be treated is changed, in other words, when the target tissue to be treated is changed from any one of the dermis, the epidermis, and the subcutis to another, the controller 6 controls the RF adjuster 2 to adjust the frequency of the RF energy. Further, when the target tissue is changed, the controller 6 receives the impedance of the tissue from the sensor 5 and controls the impedance matcher 3.

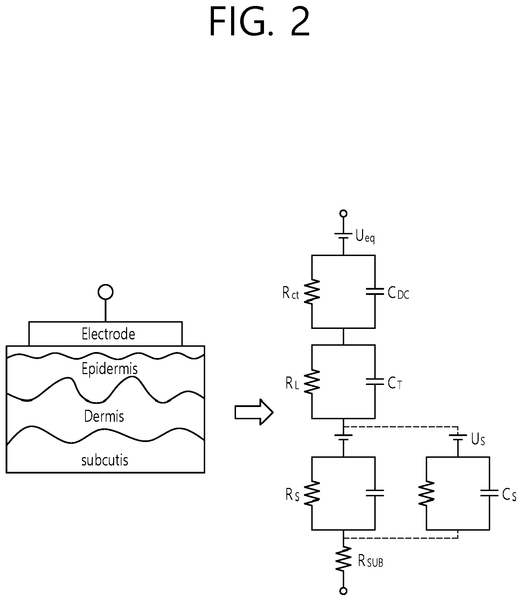

FIG. 2 shows a schematic diagram of skin tissues and a circuit diagram approximating the electrical characteristics of the tissues.

Referring to FIG. 2, the circuit diagram is approximated by mirroring the electrical characteristics of the skin tissue when electric energy is transferred to the surface of skin through the electrode being in close contact with the surface of skin. Here, U_{eq} is the electric potential of the electrode, Uₛ is the electric potential of the skin, R_{ct} is the charge transfer resistance at an interface, C_{DC} is double layer capacitance, R_{L} is the resistance of electrolyte and lead, C_{T} is a strong capacitive behavior at a skin-electrode interface due to the lack of hydrogel, Rₛ and Cₛ are the resistance and capacitance of stratum corneum, and R_{SUB} is the resistance of subcutis.

Although such electrical approximation is carried out, the electrical characteristics of the skin tissue are varied depending on which one is selected from among epidermis, dermis, and subcutis as the tissue to which the RF energy is mainly transferred, and thus an adjustment value for the impedance matching is also varied. For example, when the epidermis is selected as a main treatment target, the electrical characteristics of the epidermis are considered to perform the impedance matching. Further, when the dermis is selected as the main treatment target, the electrical characteristics of both the epidermis and the dermis are taken into account to carry out the impedance matching. Furthermore, when the subcutis is selected as the main treatment target, the electrical characteristics of all the epidermis, the dermis, and the subcutis are considered to perform the impedance matching.

However, the adjustment values for the impedance matching may be largely varied depending on parts, individuals, and conditions of tissue, and thus detailed descriptions of specific numerical values will be omitted.

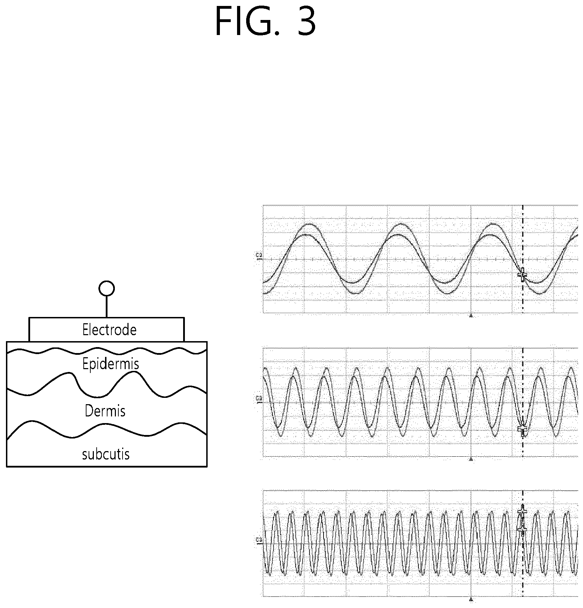

FIG. 3 is a conceptual diagram of RF energy's frequency selected for each target tissue.

FIG. 3 shows the frequencies and electric potential selected when the RF energy is applied to one selected from among the epidermis, the dermis, and the subcutis, and the current values of when the RF energy is applied to the tissue without the impedance matching. It is known that the area to which the RF energy is applied is formed shallow and wide in tissue when the frequency is low within a frequency range of 2 to 20 MHz, but formed deep and narrow when the frequency is high.

Therefore, the controller 6 may control the RF adjuster 2 to adjust the frequency of the RF energy into 2.25 MHz when the epidermis is the target tissue to which the RF energy is transferred, 6.75 MHz when the dermis is the target tissue, and 13.56 MHz when the subcutis is the target tissue.

Further, the controller identifies the main RF-energy applying area of the tissue based on the frequency of the applied RF energy, and controls the impedance matcher 3 to perform the impedance matching because the impedance of the tissue value is varied at this time.

According to the first embodiment of the disclosure, the frequency of the RF energy is adjusted according to which tissue is selected as the target tissue, and the impedance matching is performed in real time as the main target tissue is changed, thereby maximizing an RF energy transfer efficiency and improving the accuracy of the treatment.

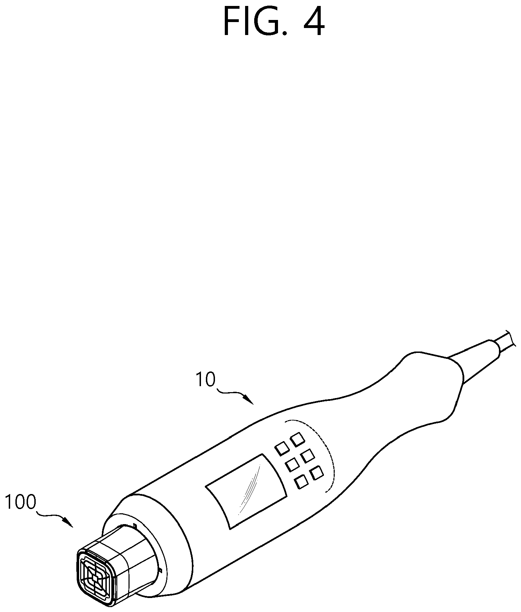

Below, a treatment apparatus using RF energy according to a second embodiment of the disclosure will be described with reference to FIGS. 4 to 7.

FIG. 4 shows a partially enlarged perspective view of a handpiece in a treatment apparatus using RF energy according to a second embodiment of the disclosure.

Referring to FIG. 4, the treatment apparatus using the RF energy according to the second embodiment of the disclosure may be a treatment apparatus for skin tissue remodeling. Specifically, the treatment apparatus using the RF energy according to the second embodiment of the disclosure may be an apparatus for treating tissue of face skin. In this embodiment, the treatment apparatus may include a handpiece 10 to be held by a user, and a tip 100 provided at one side of the handpiece 10. A plurality of electrodes that can come into close contact with skin may be provided at the end portion of the tip 100. A user can select a treatment area by moving the position of the handpiece 10.

Meanwhile, according to this embodiment, an RF generator, an RF adjuster, an impedance matcher, a sensor, and a controller may be provided like those of the foregoing embodiment, but these elements may be placed in a main body provided separately from the foregoing handpiece.

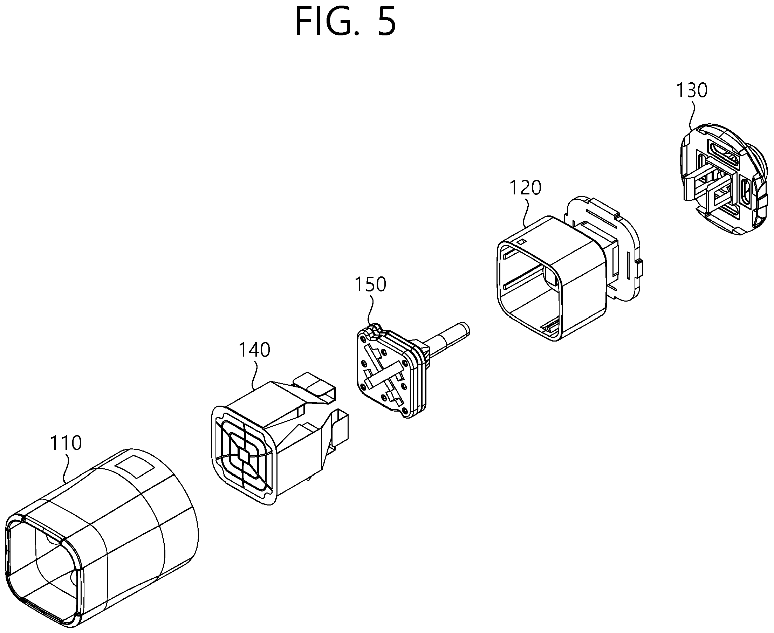

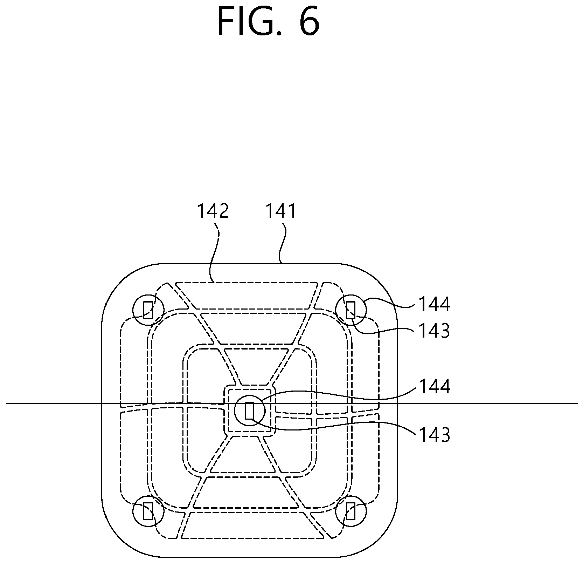

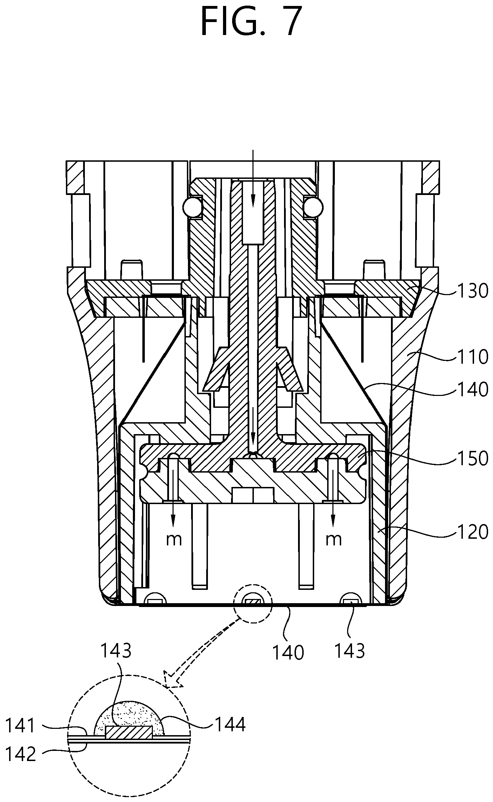

FIG. 5 is an exploded perspective view of the tip shown in FIG. 4, FIG. 6 is a plan view of the electrode module, and FIG. 7 is a cross-sectional view of the tip shown in FIG. 4.

Referring to FIGS. 5 to 7, the tip in this embodiment may include a front cover 110, an electrode module 140, a cooling channel block 150, an inner cover 120, and a rear cover 130.

The front cover 110 and the inner cover 120 are coupled to each other and configured to support the electrode module 140. The cooling channel block 150 may be disposed in the inner cover 120. The electrode module 140 includes a substrate 141, a plurality of electrodes 142. The substrate 141 is configured so that the electrodes 142 and a temperature sensor 143 can be provided on the substrate 141. The plurality of electrodes 142 may include a plurality of pairs of electrodes facing toward outside of the tip 100 as described above. The plurality of electrodes 242 may be respectively disposed in a region divided by at least one sinusodial wave. In addition, the electrodes have different distances from the center of the substrate and may be disposed in regions separated by concentric paths.

The temperature sensors 143 are provided on a substrate 141 of the electrode module opposite to the electrodes. One surface of the temperature sensor 143 may be in contact with the electrode. The temperature sensors 143 are covered by a shield 144 to prevent direct cooling by a cooling medium. The temperature sensors are configured to measuring the temperature of the electrode 142 directly. The temperature sensors may be positioned at several points to measure the temperature of the electrode where the edge effect occurs and edge effect does not occur. In FIG. 6, the temperature sensor 143 at the center measures the temperature of the electrode where the edge effect does not occur, and the temperature sensors at the outside are provided to measure the temperature of the electrode where the edge effect occurs. However, the number of such sensors in FIG.6 is only an example and may be applied to various numbers for measuring the temperature of an electrode generating an edge effect and an electrode not generating an edge effect.

The cooling channel block 150, of which one side penetrates the rear cover 130, is configured to spray a refrigerant introduced from a cooler provided in the main body toward the rear side of the electrode module 140. In this case, the cooling channel block 150 is formed with a plurality of outlets so that the electrode module 140 can evenly absorb heat in a plurality of regions, and configured so that a refrigerant m can be branched inside the cooling channel block 150 and sprayed toward the rear side of the electrode.

Meanwhile, although it is not shown, a cooler may be provided in the main body, and include a container to accommodate a cooling medium, and a cooling driver to drive the container to cause the cooling medium to flow out. For example, the cooler may be configured to cause a cryogen or the like refrigerant to flow to the cooling channel block 150, and be finally sprayed toward the rear side of the electrode module 140, thereby cooling the electrodes while evaporating.

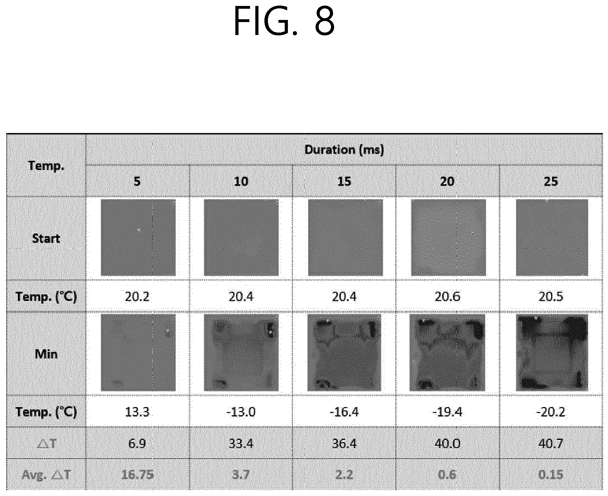

FIG. 8 shows information about heat maps and temperatures when the surface of the tip shown in FIG. 4 is cooled.

Referring to FIG. 8, a final temperature corresponding to a spraying time is shown when the refrigerant is sprayed to the rear side of the electrode module 140 by operation of the cooler. As an initial temperature, the temperature of the electrode was set to a room temperature of 20 to 21°C. The refrigerant was sprayed for spraying duration set in units of 5 seconds from 5 to 25 seconds. As the spraying duration of the refrigerant increases, the final temperature was low. In this case, it is noted that the rear side of the electrode module 140 is uniformly cooled through a spraying hole formed in the cooling channel block 150 and thermal distribution is uniformly formed at a central portion.

As described above, the cooler can cool the rear side of the electrode. In this case, electrically capacitive coupling is formed between the electrode and the skin, and therefore the cooler operates to lower the temperature of the skin surface when the surface of skin is excessively increased in temperature by the transfer of the RF energy. At this time, a temperature sensor provided at one side of the tip may be used to measure the temperature on the surface of the skin being in close contact with the tip, so that the measured temperature can be fed back and used in controlling the cooler.

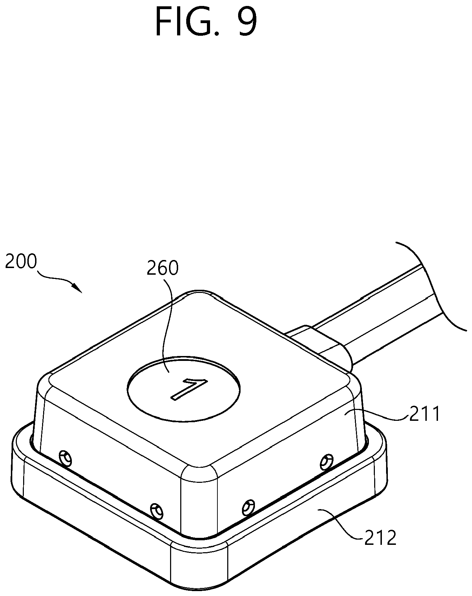

Below, a treatment apparatus using RF energy according to a third embodiment of the disclosure will be described with reference to FIGS. 9 to 12.

The treatment apparatus using the RF energy according to the third embodiment may include a plurality of body applicators with respect to one main body. Each body applicator may be attached to a human body and configured to transfer the RF energy to the human body. In the third embodiment, the body applicator applies the RF energy to break down fat cells, thereby finally exhibiting a body contouring effect.

FIG. 9 shows a partially enlarged perspective view of the body applicator in the treatment apparatus using the RF energy according to the third embodiment of the disclosure.

Referring to FIG. 9, the body applicator is shown. A plurality of body applicators may be provided to perform overall treatment for an abdomen having a relatively large area at once, and the plurality of body applicators may be used at a time. Each body applicator has a predetermined size, and may be configured to transfer the RF energy to skin, for example, the abdomen while being attached to the skin.

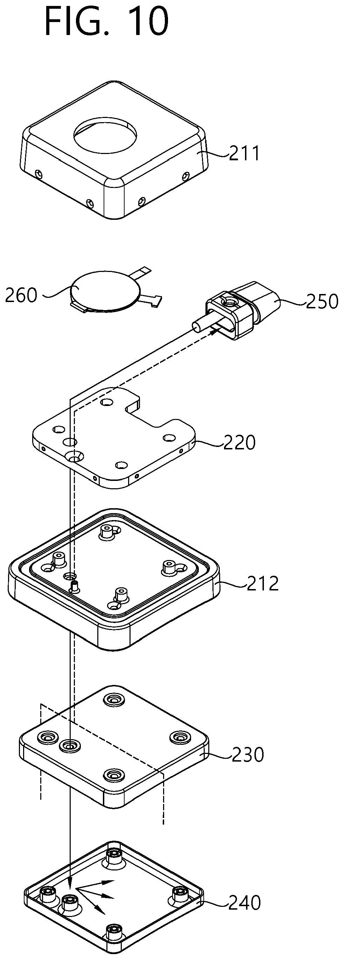

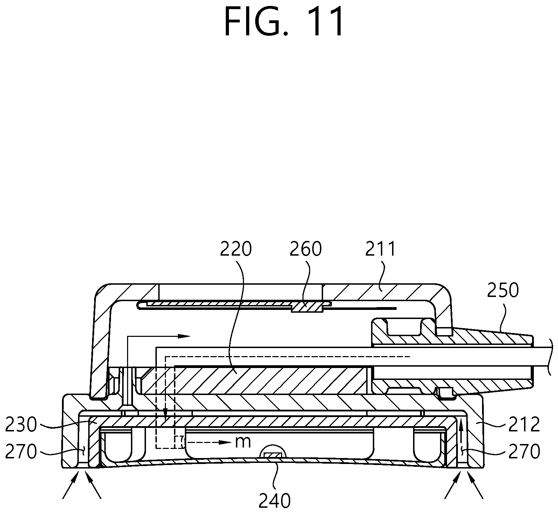

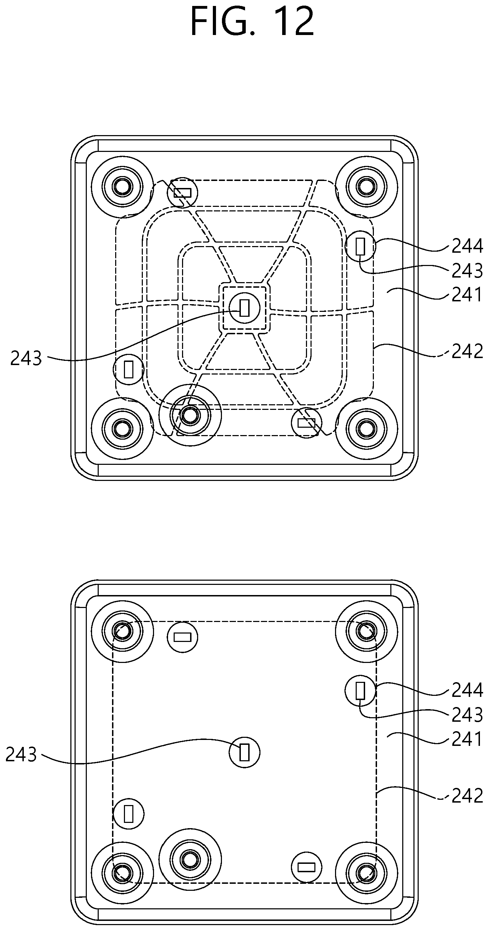

FIG. 10 shows an exploded perspective view of the body applicator shown in FIG. 9, FIG. 11 shows a cross-sectional view of the body applicator shown in FIG. 9, and Fig. 12 shows a plan view of an electrode plate and a view showing another embodiment.

Referring to FIGS. 10 to 12, a body applicator 200 may include an upper outer cover 211, a lower outer cover 212, an insulator 220, an inner cover 230, an electrode plate 240, a connector 250, and a display 260.

The upper outer cover 211 and the lower outer cover 212 may be coupled in up and down directions, while forming a space therebetween. In the space between the upper outer cover 211 and the lower outer cover 212, the insulator 220 is provided to prevent interference between an electrical path and a negative pressure path (to be described later).

The lower outer cover 212 is opened downward, and configured to accommodate the inner cover 230 and the electrode plate therein. The lower outer cover 212 may be internally provided with the inner cover 230 being spaced apart from the inner side of the lower outer cover 212. In other words, a predetermined gap may be formed between the inner side of the lower outer cover 212 and the outer side of the inner cover 230. Such a gap may function as passage negative pressure.

The electrode plate 240 may include a substrate 241, a plurality of electrodes 242 and temperature sensors 243. The substrate 241 is provide with flat shape. The plurality of electrodes may be formed having a predetermined pattern on the lower side of the electrode plate 240 in shown upper side of FIG 12. The temperature sensors 243 are provided on a substrate 241 of the electrode module opposite to the electrodes. One surface of the temperature sensor 243 may be in contact with the electrode. The temperature sensors 243 are covered by a shield 244 to prevent direct cooling by a cooling medium. The temperature sensors are configured to measuring the temperature of the electrode 242 directly. The temperature sensors may be positioned at several points to measure the temperature of the electrode where the edge effect occurs and edge effect does not occur. However, in this embodiment, the shape of the electrode is not limited to the above-described shape, and may be deformed into a shape such as a single plate as shown lower in FIG. 12. Wires respectively connected to the electrodes may pass through the inner cover and be connected in a space between the upper outer cover 211 and the lower outer cover 212.

The connector 250 is provided at one side of the upper outer cover 211, electrically connected to the main body, and configured to allow a tube and a wire for transferring the RF energy to pass therethrough. The tube may receive negative pressure from a vacuum pump of the main body, and fluid-communicate with a gap 270.

The gap 270 receives negative pressure through the tube as the vacuum pump provided in the main body operates, and forms the negative pressure along a circumferential direction of the electrode plate 240, thereby allowing the electrode plate 240 to come into close contact with skin.

The display 260 is provided on an upper side of the upper outer cover 211 and configured to display information, for example, the unique information about each of the plurality of body applicators 200.

In this embodiment, the flow path for suction and the flow path for spraying the cooling media may be independently provided. A flow path for spraying the cooling media is formed while passing through the connector 250, the upper outer cover 211, the lower outer cover 212, the insulator 220, and the inner cover 230. Accordingly, the cooling medium introduced through the connector 250 may be sprayed toward the electrode plate 240 from the inside of the applicator 200. At this time, since the cooling passage is provided separately from the channel for vacuum, even if the cooling medium is sprayed, the vacuum environment is not affected.

Therefore, in the present embodiment, even if an edge effect is occurred when RF energy is transmitted, it can be detected in real time and can be cooled quickly.

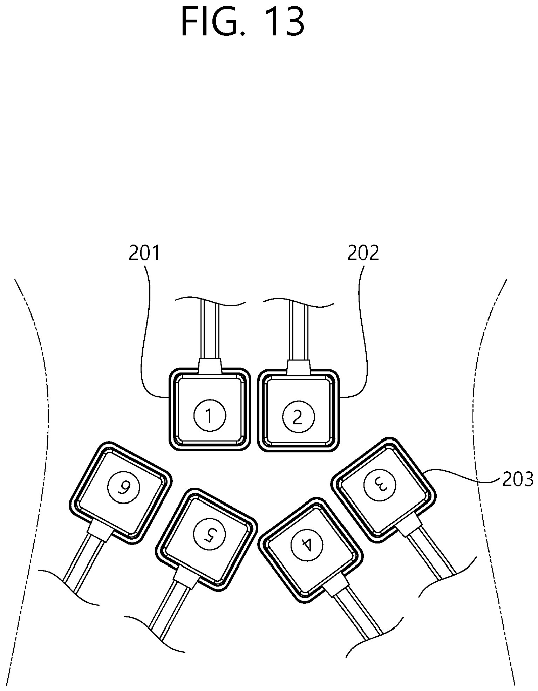

FIG. 13 shows a use state of the treatment apparatus using the RF energy according to the third embodiment of the disclosure.

Referring to FIG. 13, the plurality of body applicators in this embodiment may be attached to skin and used together at a time. For example, six body applicators may be provided and assigned with serial numbers, thereby being attached to parts of the abdomen.

In this embodiment, the controller is configured to control the body applicators independently of each other.

The controller may divide the whole treatment area into sub treatment areas, and control the divided areas to sequentially undergo the treatment. For example, any one of the body applicators, in which the treatment sequence is performed, may apply the RF energy by setting the frequency of the RF energy in order of 2.25, 6.75 and 13.56 MHz. In other words, the RF energy having the frequency of 2.25 MHz is applied to perform preheating throughout a wide area during a first treatment time, the RF energy having the frequency of 6.75 MHz is then applied during a second treatment time, and the RF energy having the frequency of 13.56 MHz is then applied during a third treatment time, thereby raising the temperature up to the treatment temperature for breaking down fat cells. In this case, each treatment time may be set in advance, or when the temperature of each tissue is measured to be higher than a threshold temperature, the corresponding treatment sequence may be terminated to perform the next treatment sequence.

In this case, the controller may apply the RF energy while interworking with an adjacent body applicator. In other words, the controller may apply the RF energy having the frequency of 2.25 MHz to a second body applicator 202 adjacent to a first body applicator 201, when the RF energy having the frequency of 6.75 MHz is applied after applying the RF energy having the frequency of 2.25 MHz to the first body applicator 201. After a predetermined period of time has elapsed, the RF energy having the frequency of 2.25 MHz is applied to a third body applicator 203 adjacent to the second body applicator 202. In this case, the controller may control the RF energy having the frequency of 6.75 MHz to be applied to the third body applicator 203, and the RF energy having the frequency of 13.56 MHz to be applied to the first body applicator 201. In other words, regarding the whole treatment areas, the area to which the RF energy having the frequency of 2.25 MHz is applied is shifted according to the serial numbers for the positions to which the body applications are attached. Similarly, the area to which the RF energy having the frequency of 6.75 MHz is applied follows the area to which 2.25 MHz is applied. Likewise, the area to which the RF energy having the frequency of 13.56 MHz is applied may follow the area to which the RF energy having the frequency of 6.75 MHz is applied.

Meanwhile, the controller may set the first treatment time, the second treatment time, and the third treatment time to be different from one another, and therefore the times at which the frequency is changed in the body applicator may be different from each other.

Meanwhile, when the frequency of the RF energy applied to each body applicator is changed, the controller may use the impedance matcher connected to each body applicator to perform the impedance matching. In this case, the impedance matching may be performed based on the impedance of the tissue, to which the RF energy is mainly applied, like that described in the first embodiment.

As described above, according to the third embodiment of the disclosure, the plurality of body applicators are provided, and the treatment is performed while sequentially changing the frequencies of the RF energy in each body applicator. In this case, when the frequency of the RF energy is changed in each body applicator, the impedance matching may be performed in real time.

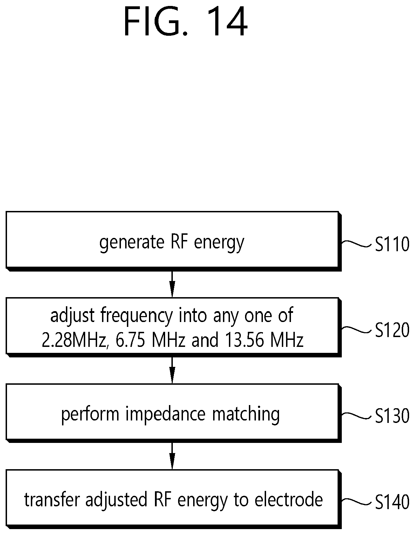

Below, a control method of a treatment apparatus using RF energy according to a fourth embodiment of the disclosure will be described with reference to FIG. 14.

FIG. 14 shows a flowchart of a control method of a treatment apparatus using RF energy according to a fourth embodiment of the disclosure.

Referring to FIG. 14, the control method of the treatment apparatus using the RF energy according to the fourth embodiment of the disclosure may include steps of generating RF energy (S110), adjusting the frequency into any one of 2.28 MHz, 6.75 MHz and 13.56 MHz (S120), performing the impedance matching (S130), and transferring the adjusted RF energy to the electrode (S140).

Step S110 of generating the RF energy corresponds to step of generating the RF energy using energy applied from a power supply.

Step S120 of adjusting the frequency into any one of 2.28 MHz, 6.75 MHz and 13.5 6 MHz corresponds to step of selecting the frequency of the RF energy so that the target tissue to be treated can be any one selected from among the epidermis, the dermis and the subcutis. The lower the frequency, the shallower and wider the area to which the RF energy is intensively applied. The higher the frequency, the deeper and narrower the area to which the RF energy is intensively applied. Therefore, this step corresponds to step of selecting the frequency by selecting a treatment area.

Step S130 of performing the impedance matching corresponds to step of performing the impedance matching because the area to which the RF energy is mainly applied is changed when the frequency of the RF energy is selected.

Step S140 of transferring the adjusted RF energy to the electrode corresponds to step of transferring the RF energy to the electrode in the state that the impedance matching with the tissue is performed when the frequency of the RF energy is changed and the RF energy having the changed frequency is transferred. When the RF energy is transferred to the electrode, deep heat is generated in the tissue as the RF energy is transferred to the tissue capacitively coupled to the electrode.

Meanwhile, although it is not shown, when the frequency of the RF energy is changed by a user or a previously input control sequence, step S120 of adjusting the frequency into any one of 2.28MHz, 6.75 MHz and 13.56 MHz to step S140 of transferring the adjusted RF energy to the electrode may be carried out again.

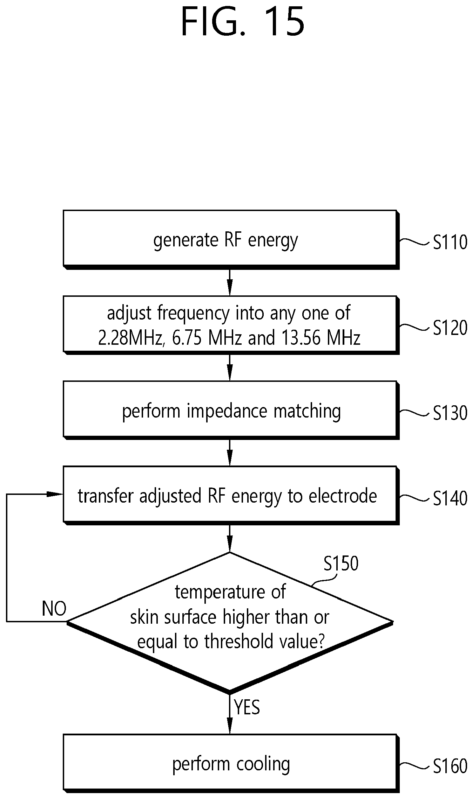

Below, a flowchart of a control method of a treatment apparatus using RF energy according to a fifth embodiment of the disclosure will be described with reference to FIG. 15.

Referring to FIG. 15, step S110 of generating the RF energy to step S140 of transferring the adjusted RF energy to the electrode in the foregoing embodiment may be equally performed in the fifth embodiment.

This embodiment may further include step S150 of identifying whether the temperature of the skin surface is higher than or equal to a threshold value and step S160 of cooling, while the treatment is performed by applying the adjusted RF energy to the tissue.

Step S150 of identifying whether the temperature of the skin surface is higher than or equal to the threshold value includes measuring the temperature of the skin surface by a temperature sensor, and identifying whether the temperature of the skin surface excessively rises above the threshold value. In this step, when the temperature measured by the temperature sensor does not exceed the threshold value, step S140 of transferring the adjusted RF energy to the electrode may be continued. On the other hand, when it is identified that the temperature of the skin surface is higher than or equal to the threshold value during step S140 of transferring the adjusted RF energy to the electrode, step S160 of cooling may be performed.

Step S160 of cooling is performed to prevent the surface of skin from being excessively damaged, by finally cooling the tissue with a cooling fluid, i.e., a refrigerant. Step S160 of cooling may be performed by cooling the rear side of the electrode being in close contact with the skin so as to lower only the temperature of the skin surface without affecting the transfer of the RF energy. In this step, the cooling medium, e.g., the cryogen may be sprayed to the rear side of the electrode so as to lower only the temperature without electrically affecting the transfer of the RF energy. The cryogen or the like refrigerant can lower the temperature of the electrode while evaporating, thereby finally lowering the temperature of the skin surface.

The fifth embodiment described above may be applied to the foregoing treatment apparatus using the RF energy according to the second embodiment.

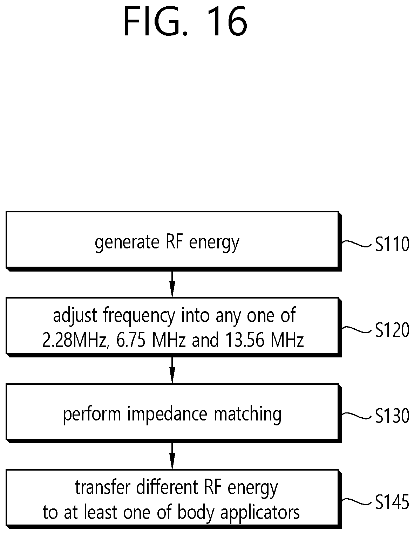

Below, a control method of a treatment apparatus using RF energy according to a sixth embodiment of the disclosure will be described with reference to FIG. 16.

FIG. 16 shows a flowchart of a control method of a treatment apparatus using RF energy according to a sixth embodiment of the disclosure.

Referring to FIG. 16, step 110 of generating the RF energy to step 130 of performing the impedance matching in the foregoing forth embodiment may be equally performed in the sixth embodiment. In the sixth embodiment, step S145 of transferring different RF energy to at least one among the body applicators may be performed after step S130 of performing the impedance matching.

Step 145 of transferring different RF energy to at least one among the body applicators corresponds to step of individually adjusting the frequency of the RF energy being transferred to the plurality of body applicators and the applying time. The plurality of body applicators attached to the treatment area, e.g., the abdomen may be controlled to sequentially apply the RF energy having the frequencies of 2.28MHz, 6.75 MHz and 13.56 MHz to each treatment area. Meanwhile, the frequency of the RF energy applied based on time may be adjusted from a low frequency to a high frequency, but the frequency to be selected may be varied depending on predetermined conditions. For example, it may be previously set to apply the RF energy having the frequency of 6.75 MHz or 13.56 MHz to a specific treatment area directly without a separate preheating process when the area to which the RF energy is initially applied is already heated up to a predetermined temperature.

Meanwhile, although it is not shown, in this embodiment, the suction power of the body applicator may be increased by restarting the vacuum pump when the temperature measured by the temperature sensor is excessively low beyond a predicted range.

The sixth embodiment described above may be applied to the foregoing treatment apparatus using the RF energy according to the third embodiment.

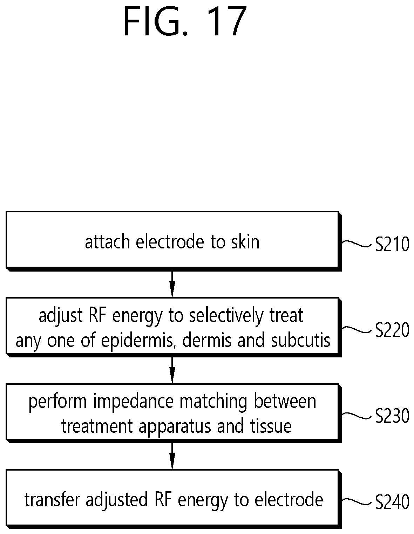

Below, a treatment method of a treatment apparatus using RF energy according to a seventh embodiment of the disclosure will be described with reference to FIG. 17.

FIG. 17 shows a flowchart of a treatment method using RF energy according to a seventh embodiment of the disclosure.

Referring to FIG. 17, the treatment method using the RF energy according to the seventh embodiment of the disclosure includes steps of attaching the electrode to skin (S210), adjusting the RF energy to selectively treat any one of the epidermis, the dermis and the subcutis (S220), performing the impedance matching between the treatment apparatus and the tissue (S230), and transferring the adjusted RF energy to the tissue (S240).

Step S210 of attaching the electrode to the skin corresponds to step of attaching the plurality of electrodes to the surface of skin for the target tissue desired to be treated.

Step S220 of adjusting the RF energy to selectively treat any one of the epidermis, the dermis and the subcutis corresponds to step of selecting the frequency of the RF energy based on a given sequence or a user' selection. In this case, the frequency of 2.25 MHz may be selected for the epidermis, the frequency of 6.75 MHz may be selected for the dermis, and the frequency of 13.56 MHz may be selected for the subcutis.

Step S230 of performing the impedance matching between the treatment apparatus and the tissue corresponds to step of performing the impedance matching between the treatment apparatus and the tissue because the impedance value is changed as the RF energy is changed in the transfer path inside the tissue when the frequency of the RF energy is changed.

Step S240 of transferring the adjusted RF energy to the tissue corresponds to step of transferring the RF energy, which is adjusted in parameters such as the frequency and the power as the impedance matching is completed, to the tissue and treating the tissue. In this step, when the RF energy is applied to the tissue, preheating may be achieved throughout a wide area (2.28 MHz), and the tissue may be heated and treated at a temperature that causes tissue remodeling in a deep part (13.56 MHz). During this step, step S230 of performing the impedance matching between the treatment apparatus and the tissue may be carried out in real time, and the impedance matching may be performed in real time by mirroring the frequency of the RF energy and the impedance of the tissue, in particular, impedance change according to the temperature of the tissue.

Meanwhile, although it is not shown, step of cutting off the RF energy may be performed when it is identified that the temperature of the target tissue reaches the treatment temperature.

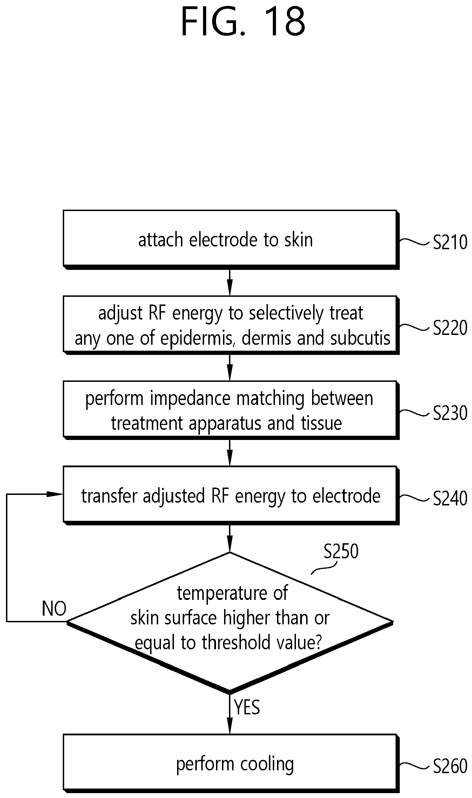

FIG. 18 shows a flowchart of a treatment method using RF energy according to an eighth embodiment of the disclosure.

Referring to FIG. 18, in this embodiment, treatment for facial skin tissue is performed.

In this embodiment, step S210 of attaching the electrode to the face may be first performed, and step S220 of adjusting the RF energy and step 230 of performing the impedance matching may be carried out like those of the seventh embodiment described above.

In this embodiment, step S250 of identifying whether the temperature of skin exceeds the threshold value and step S260 of cooling the surface of skin may be performed while performing step S240 of transmitting the adjusted RF energy to the tissue.

Step S250 of identifying whether the temperature of skin exceeds the threshold value corresponds to step of measuring the temperature to prevent the surface of skin from being excessively damaged by the transfer of the RF energy. In this step, the temperature sensor provided adjacent to the electrode may for example be used to measure the temperature. When the temperature of the skin surface to which the RF energy is applied does not exceeds the threshold value, step S240 of transferring the RF energy to the tissue may be continuously performed.

When it is identified in step S250 that the temperature of skin exceeds the threshold value, step S260 of cooling may be performed. In step S260 of cooling, the electrode is cooled, thereby ultimately lowering the temperature of the skin surface. Step S260 of cooling may be terminated when the temperature of the skin surface is lowered below the threshold value. Meanwhile, step S240 of transferring the RF energy to the tissue is continued even while step S250 of identifying whether the temperature of skin exceeds the threshold value and step S260 of cooling are performed. Therefore, the temperature of a deep part continuously rises up to the treatment temperature, or the treatment temperature is maintained.

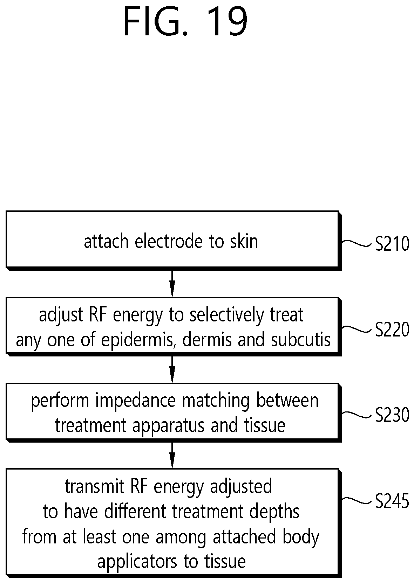

FIG. 19 shows a flowchart of a treatment method using RF energy according to a ninth embodiment of the disclosure.

Referring to FIG. 19, the ninth embodiment of the disclosure may be implemented using a plurality of body applicators capable of treating an abdomen.

In this the embodiment, the electrode may be attached to abdominal skin in step S210 of attaching the electrode to the skin. Then, step S220 of adjusting the RF energy to selectively treat any one of the epidermis, the dermis and the subcutis and step 230 of performing the impedance matching between the treatment apparatus and the tissue may be carried out like those of the seventh embodiment described above.

In this embodiment, step S245 of transmitting the RF energy adjusted to have different treatment depths from at least one among the attached body applicators to the tissue may be performed to treat the tissue.

In this step S245, a heating area are varied in range and depth depending on the frequencies of the RF energy, and therefore the plurality of attached body applicators are prioritized and sequentially perform the treatments in specific sequence. As an example, first, the frequency of RF energy at which energy is concentrated on the epidermis may be selected, and the treatment position may be moved along the skin surface direction. Next, after adjusting the frequency of the RF energy so that the energy can be concentrated in the dermis, the treatment position can be moved along the skin surface direction. Next, similarly to the treatment of the fat layer, the frequency of the RF energy may be adjusted so that the RF energy can reach the fat layer and be heated, and then the treatment position may be moved along the skin surface direction.

Meanwhile moving the aforementioned treatment position along the skin surface direction means that the treatment position changes along the skin surface direction. This can be achieved, for example, by sequentially delivering RF energy to body applicators #1, #2, and #3, ie, swapping, when body applicators #1, #2 and #3 are attached to the skin.

As described above, there are provided a treatment apparatus using RF energy according to the disclosure, a control method thereof, and a treatment method using the same, in which the RF energy is adjusted according to penetration depths from the surface of skin, thereby having an effect on improving treatment accuracy. Further, impedance matching is performed according to the penetration depths of the RF energy, thereby having an effect on maximizing a treatment effect in a treatment area.

## Claims

1. A treatment apparatus using radio frequency (RF) energy, comprising:
an RF generator (1) configured to generate the RF energy;
an RF adjuster (2) configured to adjust the RF energy;
electrodes (4) configured to noninvasively transfer the adjusted RF energy to a target tissue while being in contact with skin; and
a controller (6) configured to control the RF generator and the RF adjuster,
**characterized in that** the controller (6) adjusts a frequency of the RF energy to intensively transfer the RF energy to any one of epidermis, dermis and subcutis.

2. The treatment apparatus of claim 1, wherein the controller (6) controls the RF adjuster to transfer the RF energy by selecting any one of three frequencies.

3. The treatment apparatus of claim 2, further comprising an impedance matcher (3) provided between the RF adjuster and the electrodes,
wherein the controller (6) controls the impedance matcher (3) to perform impedance matching between the target tissue and the RF adjuster (2) when the RF adjuster (2) adjusts the frequency of the RF energy.

4. The treatment apparatus of claim 3, wherein the controller (6) controls the RF adjuster (2) to select the frequency of the RF energy from among 2.28 MHz, 6.75 MHz and 13.56 MHz.

5. The treatment apparatus of claim 4, wherein the electrodes (4) are provided at an end portion of a handpiece (100) for face treatment.

6. The treatment apparatus of claim 5, wherein the controller (6) controls the RF adjuster (2) to change the frequency of the RF energy in order of 2.25 MHz, 6.75 MHz and 13.56 MHz.

7. The treatment apparatus of claim 6, further comprising a cooler configured to cool at least one of the electrodes,
wherein the controller (6) controls the cooler to operate when a temperature of a skin surface rises above a threshold value.

8. The treatment apparatus of claim 7, further comprising temperature sensors configured to directly measure the temperature of at least one of the electrodes (4) .

9. The treatment apparatus of claim 8, wherein the temperature sensors are configured to measure temperature of a plurality of points including at least the area where the edge effect occurs while the RF energy is transferred.

10. The treatment apparatus of claim 4, wherein the electrodes (4) are provided in a body applicator (200) attachable to a human body.

11. The treatment apparatus of claim 8, wherein the electrodes (4) are provided on a substrate, and disposed in a region divided by at least one sinusoidal wave line.

12. The treatment apparatus of claim 11, wherein
the body applicator (200) provided in plurality, and
the controller (6) controls the RF adjuster (2) to adjust the frequency of the RF energy, for at least one body applicator (200), according to predetermined conditions.

13. The treatment apparatus of claim 12, wherein the controller (6) controls the RF adjuster (2) to adjust the frequency of the RF energy, for the body applicator (200), in order of 2.25 MHz, 6.75 MHz and 13.56 MHz.

14. The treatment apparatus of claim 14, wherein
the plurality of body applicators (200) are assigned with unique numbers, respectively, and
the controller (6) controls the RF adjuster (2) to adjust the frequency of the RF energy in sequence of the body applicators (200) according to the unique numbers.

15. The treatment apparatus of claim 11, further comprising
a vacuum pump, and
a passage provided in the body applicator (200) and configured to fluid-communicate with the vacuum pump,
wherein the passage is formed along the periphery of the electrode, and is configured so that the body applicator can be fixed to the human body while RF energy is transmitted.
